# EUROPEAN PATENT SPECIFICATION

(11) **EP 2 786 737 B1**
(45) Date of publication and mention of the grant of the patent: **19.04.2017**
(21) Application number: 12853478.1
(22) Date of filing: 28.11.2012
(51) Int. Cl.: A61K 8/19, A61K 8/365, A61K 47/02, A61K 47/04, A61K 47/14, A61Q 19/00, A61Q 19/10, C02F 1/66, C02F 1/68, C02F 1/70

(54) **REDUCING AGENT CONTAINING SODIUM BOROHYDRIDE**
REDUKTIONSMITTEL MIT NATRIUMBORHYDRID
AGENT RÉDUCTEUR CONTENANT DU BOROHYDRURE DE SODIUM

(30) Priority: 29.11.2011 JP 2011259708
(43) Date of publication of application: 08.10.2014
(73) Proprietor: Shibano, Tetsuya, Osaka-shi, Osaka 546-0042 (JP)
(72) Inventor: TANAKA, Sei, Osaka-shi Osaka 552-0007 (JP)
(74) Representative: Hering, Hartmut
(86) International application number: PCT/JP2012/080784
(87) International publication number: WO 2013/081023

(56) References cited:
- EP-A2- 1 369 947
- WO-A1-2007/055146
- WO-A1-2010/056742
- JP-A- 2008 307 051
- JP-A- 2010 275 510
- US-A- 5 931 172

## Description

### TECHNICAL FIELD

The present invention relates to a bath additive, an external preparation for skin, a reducing agent for water, a method for producing a reduced water, a reduced water, and a method for reducing an object.

### BACKGROUND ART

Conventional bath additives include those for adding scent and color to hot water to provide pleasure and relaxation for a bathing person, and those that generate carbon dioxide gas for enhancing blood circulation (e.g., cf. Patent Document 1).

### CITATION LIST

### [PATENT DOCUMENTS]

[PATENT DOCUMENT 1] Japanese Laid-Open Utility Model Publication No. 3-111348

### SUMMARY OF THE INVENTION

### PROBLEMS TO BE SOLVED BY THE INVENTION

However, enhancement of blood circulation by carbon dioxide gas cannot provide an advantageous effect large enough to maintain health by itself.

Therefore, an objective of the present invention is to provide a bath additive, an external preparation for skin, a reducing agent for water, a method for producing a reduced water, a reduced water, and a method for reducing an object, each of which prevents aging and provides excellent advantageous effects in health maintenance and cosmetic advantageous effects.

### SOLUTION TO THE PROBLEMS

In order to achieve the aforementioned objective, a bath additive according to the present invention contains at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent.

Sodium borohydride used as a hydrogen generating agent reacts more rapidly in a stronger acidic condition to generate a large quantity of hydrogen. However, in such cases, although hydrogen is generated rapidly in large quantity, the generated hydrogen molecules (H₂) in the form of gaseous hydrogen is mostly released in air without being dissolved in bath water. Thus, time is required for the oxidation-reduction potential (ORP value) of the bath water to become lower, and the generation of hydrogen cannot be sustained for a long period of time.

According to the aforementioned characteristic configuration, the first and second alkaline agents are contained. These alkaline agents make the bath water alkaline. In addition, the first alkaline agent generates carbon dioxide gas (CO₂) in the bath water when being dissolved. When the carbon dioxide gas is generated, the bath water is stirred, and the gaseous hydrogen (hydrogen molecules, H₂) generated from sodium borohydride in a minute amount is efficiently dissolved and dispersed in the bath water. Thus, the oxidation-reduction potential of the bath water can be rapidly set in a reduction state (lowered).

Furthermore, the second alkaline agent stabilizes and maintains the basicity of the bath water. With this, since the bath water is stably maintained in alkaline for a long period of time, rapid generation of gaseous hydrogen by sodium borohydride is suppressed. In addition, since the generated gaseous hydrogen is produced in a minute amount as fine air bubbles, the gaseous hydrogen is easily dissolved in the bath water. As a result, generation of hydrogen from sodium borohydride is sustained for a long period of time, and the oxidation-reduction potential of the bath water can be maintained in a reduction state for a long period of time because of the dissolved hydrogen.

As mentioned above, reaction of sodium borohydride proceeds more rapidly in a more acidic condition. Since the skin of a human body is weakly acidic, more of the sodium borohydride can be caused to react in the vicinity of the skin of the person's body who is bathing, and oxidization of cells inside the body of the bathing person can be efficiently alleviated.

In the aforementioned characteristic configuration, the second alkaline agent may be at least one type selected from the group consisting of potassium citrate and sodium citrate. Since these compounds maintain the basicity of the bath water more stably, the oxidation-reduction potential of the bath water can be maintained in a reduction state for a longer period of time.

Furthermore, sodium sulfate may be further contained as an extender. In such a case, preferably, the first alkaline agent is sodium bicarbonate, the second alkaline agent is potassium citrate, the sodium bicarbonate is contained by 35 to 45 wt%, the potassium citrate is contained by 3 to 10 wt%, the sodium sulfate is contained by 50 to 60 wt%, and the sodium borohydride is contained by 0.0025 to 10 wt%. With the above described numeric value ranges, the oxidation-reduction potential of the bath water can be more stably maintained in a reduction state for a long period of time.

In order to achieve the aforementioned objective, an external preparation for skin according to the present invention contains at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent.

Sodium borohydride used as a hydrogen generating agent reacts more rapidly in a stronger acidic condition to generate a large quantity of hydrogen. However, in such cases, although hydrogen is generated rapidly in large quantity, the generated hydrogen molecules (H₂) in the form of gaseous hydrogen is mostly released in air without being dissolved in water. Thus, time is required for the oxidation-reduction potential (ORP value) of the water to become lower, and the generation of hydrogen cannot be sustained for a long period of time.

According to the aforementioned characteristic configuration, the first and second alkaline agents are contained. These alkaline agents make the water alkaline. In addition, the first alkaline agent generates carbon dioxide gas (CO₂) in the water when being dissolved. When the carbon dioxide gas is generated, the water is stirred, and the gaseous hydrogen (hydrogen molecules, H₂) generated from sodium borohydride in a minute amount is efficiently dissolved and dispersed in the water. Thus, the oxidation-reduction potential of the water can be rapidly set in a reduction state (lowered).

Furthermore, the second alkaline agent stabilizes and maintains the basicity of a solution. With this, since the solution is stably maintained in alkaline for a long period of time, rapid generation of hydrogen gas by sodium borohydride is suppressed. In addition, since the generated gaseous hydrogen is produced in a minute amount as fine air bubbles, the gaseous hydrogen is easily dissolved in the water. As a result, generation of hydrogen from sodium borohydride is sustained for a long period of time, and the oxidation-reduction potential of the water can be maintained in a reduction state for a long period of time because of the dissolved hydrogen.

As mentioned above, reaction of sodium borohydride proceeds more rapidly in a more acidic condition. Since the skin of a human body is weakly acidic, more of the sodium borohydride can be caused to react at the skin of the human body, and oxidization of cells inside the body of the bathing person can be efficiently alleviated.

In the aforementioned characteristic configuration, the second alkaline agent may be at least one type selected from the group consisting of potassium citrate and sodium citrate. Since these compounds can maintain the basicity of the water more stably, the oxidation-reduction potential of the water can be maintained in a reduction state for a longer period of time.

Furthermore, sodium sulfate may be further contained as an extender. In such a case, preferably, the first alkaline agent is sodium bicarbonate, the second alkaline agent is potassium citrate, the sodium bicarbonate is contained by 35 to 45 wt%, the potassium citrate is contained by 3 to 10 wt%, the sodium sulfate is contained by 50 to 60 wt%, and the sodium borohydride is contained by 0.0025 to 10 wt%. With the above described numeric value ranges, the oxidation-reduction potential of the water can be more stably maintained in a reduction state for a long period of time.

In order to achieve the aforementioned objective, a reducing agent for water according to the present invention contains at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent.

Sodium borohydride used as a hydrogen generating agent reacts more rapidly in a stronger acidic condition to generate a large quantity of hydrogen. However, in such cases, although hydrogen is generated rapidly in large quantity, the generated hydrogen molecules (H₂) in the form of gaseous hydrogen is mostly released in air without being dissolved in water. Thus, time is required for the oxidation-reduction potential (ORP value) of the water to become lower, and the generation of hydrogen cannot be sustained for a long period of time.

According to the aforementioned characteristic configuration, the first and second alkaline agents are contained. These alkaline agents make the water alkaline. In addition, the first alkaline agent generates carbon dioxide gas (CO₂) in the water when being dissolved. When the carbon dioxide gas is generated, the water is stirred, and the gaseous hydrogen (hydrogen molecules, H₂) generated from sodium borohydride in a minute amount is efficiently dissolved and dispersed in the water. Thus, the oxidation-reduction potential of the water can be rapidly set in a reduction state (lowered).

Furthermore, the second alkaline agent stabilizes and maintains the basicity of a solution. With this, since the solution is stably maintained in alkaline for a long period of time, rapid generation of hydrogen gas by sodium borohydride is suppressed. In addition, since the generated gaseous hydrogen is produced in a minute amount as fine air bubbles, the gaseous hydrogen is easily dissolved in the water. As a result, generation of hydrogen from sodium borohydride is sustained for a long period of time, and the oxidation-reduction potential of the water can be maintained in a reduction state for a long period of time because of the dissolved hydrogen.

In the aforementioned characteristic configuration, the second alkaline agent may be at least one type selected from the group consisting of potassium citrate and sodium citrate. Since these compounds can maintain the basicity of the water more stably, the oxidation-reduction potential of the water can be maintained in a reduction state for a longer period of time.

In order to achieve the aforementioned objective, a method for producing a reduced water according to the present invention includes adding, to water, a reducing agent containing at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent. In such a case, the second alkaline agent may be at least one type selected from the group consisting of potassium citrate and sodium citrate.

In order to achieve the aforementioned objective, a reduced water according to the present invention contains at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent. In such a case, the second alkaline agent may be at least one type selected from the group consisting of potassium citrate and sodium citrate.

In order to achieve the aforementioned objective, a method for reducing an object according to the present invention includes, adding, to water in which a reduction target object is immersed, a reducing agent containing at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent, or immersing the reduction target object in water having added therein the reducing agent to cause the reduction target object to be in a reduction state.

Sodium borohydride used as a hydrogen generating agent reacts more rapidly when the condition is more acidic to generate a large quantity of hydrogen. However, when the acidity is strong, although hydrogen is generated rapidly in large quantity, the generated hydrogen molecules (H₂) in the form of gaseous hydrogen is mostly released in air without being dissolved in water. Thus, although time is required for the oxidation-reduction potential of the water to become lower, the generation of hydrogen cannot be sustained for a long period of time. According to the aforementioned characteristic configuration, the first and second alkaline agents are contained. These alkaline agents set the bath water to be weakly alkaline. In addition, the first alkaline agent generates carbon dioxide gas (CO₂) in the water when being dissolved. When the carbon dioxide gas is generated, the water is stirred, and the gaseous hydrogen (hydrogen molecules, H₂) generated from sodium borohydride in a minute amount is efficiently dissolved and dispersed in the water. Thus, the oxidation-reduction potential (ORP value) of the water can be rapidly set in a reduction state (lowered).

Furthermore, the second alkaline agent stabilizes and maintains the basicity of the bath water. With this, since the water is maintained to be weakly alkaline for a long period of time, rapid generation of hydrogen gas by sodium borohydride is suppressed. In addition, since the generated gaseous hydrogen is produced in a minute amount as fine air bubbles, the gaseous hydrogen is easily dissolved in the water. As a result, generation of hydrogen from the sodium borohydride can be sustained for a long period of time, and the oxidation-reduction potential of the water can be maintained in a reduction state for a long period of time because of the dissolved hydrogen.

Then, by adding a reducing agent containing the aforementioned components in water in which an object is immersed, the oxidation-reduction potential of the water can be rapidly set in a reduction state (lowered), and a reduced water maintaining the reduction state for a long period of time is obtained. As a result, the water reduces an oxidation state of the object to a reduction state.

In the aforementioned characteristic configuration, the second alkaline agent may be at least one type selected from the group consisting of potassium citrate and sodium citrate. Since these compounds can maintain the basicity of the water more stably, the oxidation-reduction potential (ORP value) of the water can be maintained in a reduction state for a longer period of time.

In the aforementioned characteristic configuration, the reduction target object is a human body. As described above, since the water with the reducing agent is in a reduction state (the oxidation-reduction potential being on the minus side), active hydrogen in the water penetrates the skin, and oxidization of cells inside the body is alleviated. Furthermore, the water is alkaline and the skin of the human body is weakly acidic. Therefore, sodium borohydride is reacted more at the skin of the human body, and it becomes possible to efficiently generate active hydrogen in the vicinity of the skin to further alleviate oxidization of cells inside the body.

In the aforementioned characteristic configuration, the reduction target object is food. Since the water with the reducing agent is in a reduction state (the oxidation-reduction potential being on the minus side), active hydrogen in the water penetrates the food, and the oxidation state of the food is lessened (reduced).

### ADVANTAGEOUS EFFECTS OF THE INVENTION

With the present invention, it is possible to set the oxidation-reduction potential of hot water in a reduction state to suppress oxidization of the skin and cells inside of the body, and prevent aging. For example, it is possible to prevent development of arteriosclerosis, cancer, wrinkles, and spots, and obtain an advantageous effect in health maintenance as well as an advantageous effect in cosmetics. Lactate can be degraded, and muscular pain can be alleviated. In addition, a skin moisturization effect is obtained, and the skin can be moistened.

### BRIEF DESCRIPTION OF THE DRAWINGS

[FIG. 1] FIG. 1 is a graph chart for describing the effect of a bath additive according to the present invention.
[FIG. 2] FIG. 2 is another graph chart for describing the effect of a bath additive according to the present invention.
[FIG. 3] FIG. 3 is a figure corresponding to FIG. 2 for showing a Comparative Example of the present invention.

### DESCRIPTION OF EMBODIMENTS

Next, the present invention will be described in further detail with reference to the accompanying drawings as appropriate.

A bath additive according to the present invention includes sodium bicarbonate (NaHCO₃) as a first alkaline agent, sodium sulfate (Na₂SO₄) as an extender, potassium citrate (K₃(C₆H₅O₇)·H₂O) as a second alkaline agent, and sodium borohydride (NaBH₄) as a hydrogen generating agent.

The bath additive, when mixed and dissolved in hot water (water) in a bath tub, changes the oxidation-reduction potential of the hot water from an oxidation state (+) to a reduction state (-).

The bath additive, when mixed with hot water, sets the oxidation-reduction potential of the hot water to -350 to -150 mV, and preferably to -350 to -250 mV. If the oxidation-reduction potential is lower than the lower limit value (when the value shifts too much on the minus side), there may be an adverse effect on the skin. If the oxidation-reduction potential is higher than the upper limit value (when the value becomes too close to the plus side), there is a possibility of not being able to sufficiently suppress oxidization of cells.

The bath additive, when mixed with hot water, utilizes the sodium borohydride as a source of hydrogen to generate hydrogen molecules (H₂) as gas as well as dissolve the hydrogen molecules in the hot water, where one portion thereof becomes active hydrogen (H⁺) to increase the dissolved amount of active hydrogen (H⁺) in the hot water to obtain an active hydrogen hot water. The bath additive sets the dissolved amount of hydrogen in the hot water to 1000 to 2000 ppb, and preferably to 1000 to 1500 ppb. If the dissolved amount of hydrogen is lower than the lower limit value, advantageous effects in anti-oxidation and moisturization of the skin cannot be obtained, and if the dissolved amount of hydrogen is higher than the upper limit value, the amount of hydrogen may become too large and may pose a danger when the bath additive is used with ill intent.

The bath additive, when mixed with hot water, sets the hot water to alkaline, i.e., the pH to 7.5 to 9.5. If the pH is higher than the upper limit value, proteins on the skin may dissolve out to cause an adverse effect on the skin, and the generation (reaction) of hydrogen by the sodium borohydride may become insufficient. If the pH is lower than the lower limit value, the generation of active hydrogen is rapidly reduced and may not be sustained for a long period of time. As a result, it may become difficult to sustain the bath water in the reduction state (the oxidation-reduction potential (ORP value) being on the minus side) for a long period of time.

The sodium bicarbonate (baking soda) is contained by 35 to 45 wt%. If the concentration of the sodium bicarbonate is lower than the lower limit value, alkalinity of the hot water may become too low or the hot water may become acidic when the bath additive is mixed in the hot water, and the generation of active hydrogen may become extremely small.

Furthermore, if the concentration of the sodium bicarbonate is higher than the upper limit value, alkalinity may become too strong, and an extremely long time may be needed for the generation (reaction) of hydrogen.

The sodium sulfate is contained by 50 to 60 wt%. If the concentration of the sodium sulfate is lower than the lower limit value, the balance with the sodium bicarbonate is lost and alkalinity becomes too strong. If the concentration of the sodium sulfate is higher than the upper limit value, the balance with the sodium bicarbonate is lost and alkalinity of the hot water may become too low or the hot water may become acidic, and the generation of active hydrogen may become extremely small.

The potassium citrate is contained by 3 to 10 wt%, and more preferably by 3 to 5 wt%. If the concentration of the potassium citrate is lower than the lower limit value, the pH becomes unstable. If the concentration of the potassium citrate is higher than the upper limit value, a large amount of expensive potassium citrate is required and (low cost) production becomes difficult.

The sodium borohydride is contained by 0.0025 to 10 wt%, and more preferably by 0.003 to 3 wt%. If the concentration of the sodium borohydride is lower than the lower limit value, the generation of active hydrogen may become extremely small. If the concentration of the sodium borohydride is higher than the upper limit value, the amount of hydrogen may become too large and may pose a danger when the bath additive is used with ill intent in a closed room.

Next, as an Example, a bath additive (hereinafter, sometimes referred to as the present agent) containing 40 wt% of sodium bicarbonate, 55 wt% of sodium sulfate, 3.5 wt% of potassium citrate, and 1.5 wt% of sodium borohydride was created. The form of the bath additive was granular with a mean particle diameter of 0.05 mm for easy dissolution.

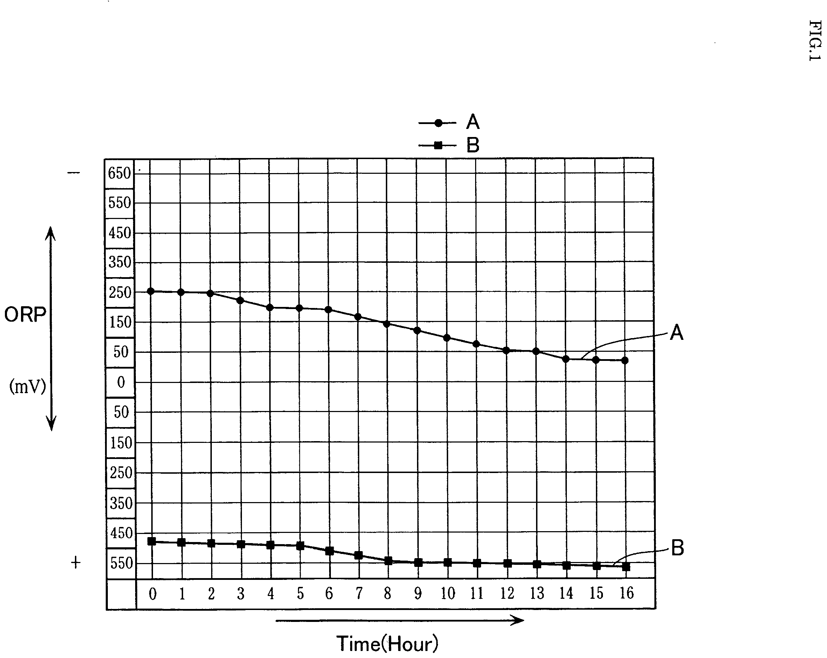

FIG. 1 is a graph chart showing each actual measurement from hot water (reference character "A" in the chart) obtained by adding 35 g of the present agent to 100 L of hot water (tap water), and hot water (reference character "B" in the chart) not having the present agent added. The horizontal axis represents elapsed time (unit: hours) of measurement and the vertical axis represents the oxidation-reduction potential (ORP value, unit: mV). It should be noted that, in the vertical axis, parts above zero indicate minus values, and parts below zero indicate plus values. With regard to the oxidation-reduction potential (ORP value), minus values indicate a reduction state, and plus values indicate an oxidation state.

As obvious from FIG. 1, when the present agent was added, a reduction state in which the oxidation-reduction potential is minus was obtained, and the reduction state was maintained. In the hot water not having the present agent added, an oxidation state in which the oxidation-reduction potential is plus was maintained from the start of the measurement, and a reduction state was never obtained.

When 40 g of the present agent was added to 100 L of hot water, the hot water's oxidation-reduction potential, which was +530 mV of an oxidation state before the adding, had changed to -300 mV (lowest value) of a reduction state after the adding.

When 40 g of the present agent was added to 200 L of hot water, the hot water's oxidation-reduction potential, which was +530 mV of an oxidation state before the adding, had changed to -170 mV (lowest value) of a reduction state after the adding.

As obvious from the above described results, it can be said that the bath additive according to the present invention had set (maintained) the oxidation-reduction potential of the hot water to the reduction state.

Furthermore, the pH of the hot water in which the present agent has been added becomes 7.5 to 9.5 (alkalinity) at which the sodium bicarbonate generates carbon dioxide gas and the sodium borohydride slowly generates active hydrogen and hydrogen without generating impurities in the weakly alkaline hot water. In addition, the dissolved amount of hydrogen in the hot water becomes 1000 to 2000 ppb.

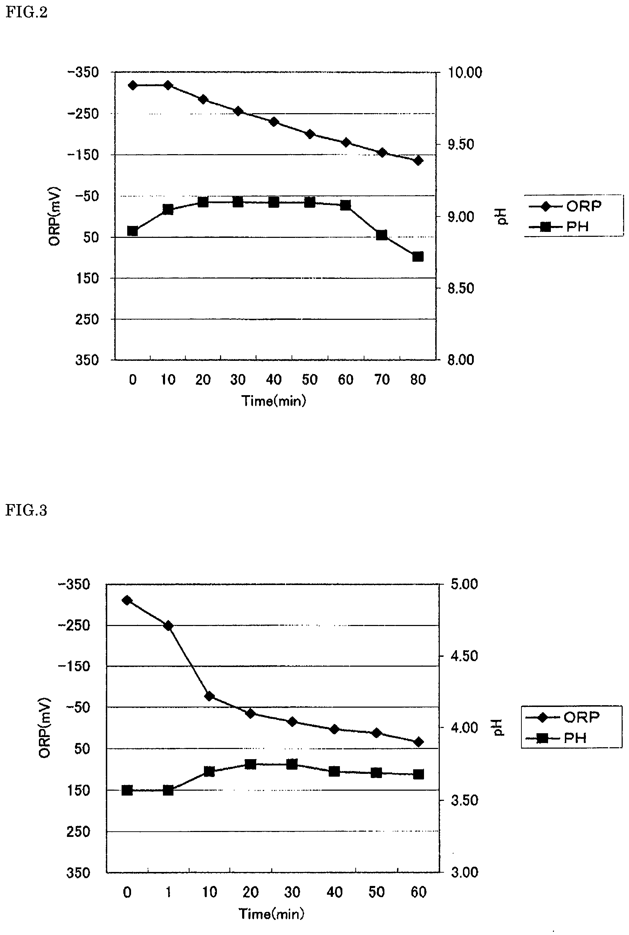

FIG. 2 is a graph of another Example in which 1.75 g of the present agent was added to 500 cc of water, and the pH and oxidation-reduction potential (ORP value, unit: mV) were measured during the course of time.

As shown in the same figure, the pH shifted to 8.9 immediately after the present agent was added. Although the pH became lower slightly thereafter, the pH was maintained around 9, and was maintained at 8.72 to be weakly alkaline even 80 minutes later. The ORP value shifted to -320 mV to a reduction state (minus) immediately after the present agent was added. Although the value increased slightly thereafter, the value was maintained in the reduction state (minus), and was maintained at -136 mV in the reduction state (minus) even 80 minutes later. In addition, minute air bubbles were generated continuously from the present agent (sodium borohydride) during the measurement.

On the other hand, as a Comparative Example, a graph is shown in which 1.75 g of the present agent and 3 g of citric acid were added to 500 cc of water, and the pH and oxidation-reduction potential (ORP value, unit: mV) were measured during the course of time.

As shown in the same figure, the pH shifted to 3.57 immediately after the present agent was added. Although the pH becomes slightly higher thereafter, the pH was maintained around 3.7 to be acidic. Although the ORP value shifted to -311mV to a reduction state (minus) immediately after the present agent was added, the value rapidly increased thereafter to be 3mV to an oxidation state (plus) 40 minutes later, and further increased. In addition, a large quantity of air bubbles was generated immediately after the adding.

As shown in FIGS. 2 and 3, with the present agent, it was possible to maintain the water (hot water) to be weakly alkaline for a long period of time. It is thought that, as a result, gaseous hydrogen was continuously generated in a minute amount as fine air bubbles from the sodium borohydride of the present agent to enable maintenance of the oxidation-reduction potential of the water in the reduction state (minus) for a long period of time.

With the reduction state of the hot water, oxidization of cells inside the body of a bathing person is alleviated, and aging is prevented. For example, occurrence of arteriosclerosis, cancer, wrinkles, and spots can be suppressed (prevented). In addition, active hydrogen (H⁺) in the hot water penetrates the skin, reacts with active oxygen (O⁻) inside the skin, and generates moisture to moisten the skin (water retention / moisturization effects are obtained). Furthermore, lactate inside of the body is degraded, and muscular pain is alleviated. In addition, blood circulation is enhanced and activational effect of the skin is enhanced by the carbon dioxide gas. It is also effective for oversensitiveness to cold and dermatitis etc.

It can be said that most (85% or higher) of the objects people ingest into the body in everyday life are oxidized. Active oxygen accumulated inside of the body keeps on oxidizing hairs, organs, and skin (epidermis), and accelerates aging. It can be said that maintenance of health and beauty is the fight against active oxygen. Accumulation of oxygen inside of the body through respiration cannot be avoided even if walking and the like to achieve health are introduced in everyday life. When a person bathes in hot water mixed with the bath additive of the present invention, oxidization is prevented, the aging process is suppressed, and both health and beauty are maintained.

It is preferable to mix 25 to 50 g of the bath additive according to the present invention with respect to 80 to 220 L of hot water. More preferably, 30 to 45 g of the bath additive according to the present invention is mixed with 80 to 120 L of hot water enough for a hip bath. It is also preferable to bathe in a hip bath for about 15 minutes for wetting (pour the hot water on) the skin with the hot water.

It should be noted that the bath additive according to the present invention may be formed as granules whose mean size is not smaller than 0.05 mm, as powder whose mean size is smaller than 0.05 mm, or, on the contrary (enlarged), as a block type or a tablet type solid form. Pigments and fragrance may also be included in the bath additive.

As described above, since the bath additive of the present invention sets concentrations of the sodium bicarbonate to 35 to 45 wt%, the sodium sulfate to 50 to 60 wt%, the potassium citrate to 3 to 10 wt%, and the sodium borohydride to 0.0025 to 10 wt%; it is possible to set the oxidation-reduction potential of the hot water in a reduction state to suppress oxidization of the skin and cells inside of the body, and prevent aging. As a result, lactate can be degraded, and muscular pain can be alleviated. In addition, it is possible to generate active hydrogen in the hot water and increase the amount thereof, and obtain a water retention (moisturization) effect of moistening the skin through generation of moisture inside the skin.

Next, a second embodiment of the present invention will be described.

Although the aforementioned first embodiment is a bath additive, the present invention can also be used as a reducing agent for water. In the second embodiment, a method for reducing an object using the reducing agent for water will be described. It should be noted that the reducing agent for water in the present embodiment is formed with components equivalent to those of the bath additive of the first embodiment.

The inventors have performed the following experiments using food (vegetables).

First, before being immersed in the reduced water produced by the water reducing agent containing the aforementioned components, each vegetable in Table 1 was crushed with a mixer, and had its ORP value measured. Next, each of the vegetables was immersed in the reduced water for 10 minutes and had its ORP value measured similarly. The reduced water was prepared by adding 20 g of the reducing agent in 5 L of water, and its ORP value after adding the reducing agent was -525 mV.

**[Table 1]**

| | ORP Value (mV) | |
|---|---|---|
| | Before adding | After adding |
| Chinese cabbage | 135 mV | -283 mV |
| Apple | 336 mV | -200 mV |
| Tomato | 159 mV | -202 mV |
| Kiwi | 58 mV | -390 mV |
| Grape | 295 mV | -370 mV |

As obvious from Table 1, it was shown that food can be set in a reduction state by only immersing the food for a predetermined time in the reduced water produced by adding the reducing agent in water. It is thought that this is a result of active hydrogen in the reduced water penetrating the food and reacting with active oxygen inside the food in a manner similar to the bath additive of the first embodiment. As described above, other than immersing the reduction target object in water having the reducing agent added therein, the reducing agent may be added to water in which the reduction target object is immersed. When the object is a human body, since the bath additive of the first embodiment functions as the reducing agent and the bath water functions as the reduced water, the invention can be phrased as a method for reducing a human body. Furthermore, the reduction target object for the reduction method according to the present invention is not limited to a human body, and can be applied to other living organisms such as mammals. For example, the present invention is applicable for health management of pets and livestock.

Next, other embodiments of the present invention will be described. Members similar to those in the aforementioned embodiments are given the same reference characters.

In each of the aforementioned embodiments, sodium bicarbonate is used as the first alkaline agent. However, the first alkaline agent is not limited thereto, and it is also possible to use at least one type selected from the group consisting of, for example, sodium bicarbonate, ammonium carbonate, and sodium carbonate. Each of these compounds sets a solution (bath water) to be weakly alkaline and generates carbon dioxide gas (CO₂) in the solution when being dissolved. As a result of generation of the carbon dioxide gas, the solution is stirred, and the gaseous hydrogen (hydrogen molecules, H₂) generated from sodium borohydride in a minute amount is efficiently dissolved and dispersed in the bath water. A substance that sets a solution to be weakly alkaline and releases carbon dioxide gas is sufficient.

In each of the aforementioned embodiments, potassium citrate is used as the second alkaline agent. However, the second alkaline agent is not limited thereto, and it is possible to use, for example, sodium citrate or at least one type selected from the group consisting of alkali metal salts or alkaline earth metal salts. Each of these compounds stably maintains basicity (weak alkalinity) of a solution (bath water). With this, since the solution is maintained to be weakly alkaline for a long period of time, rapid generation of hydrogen gas by sodium borohydride is suppressed. In addition, since the generated gaseous hydrogen is produced in a minute amount as fine air bubbles, the gaseous hydrogen is easily dissolved in the solution. As a result, generation of hydrogen from the sodium borohydride can be sustained for a long period of time, and the oxidation-reduction potential (ORP value) of the solution (bath water) can be maintained in a reduction state (minus) for a long period of time because of the dissolved hydrogen. A substance that stably maintains a solution to be weakly alkaline is sufficient.

### [Other embodiments]

The present invention can be used as an external preparation for skin. In this case, when at least one type of a first alkaline agent selected from the group consisting of sodium bicarbonate, ammonium carbonate, and sodium carbonate, at least one type of a second alkaline agent selected from the group consisting of alkali metal salts or alkaline earth metal salts, and sodium borohydride as a hydrogen generating agent are contained in the preparation; more of the sodium borohydride can be caused to react at the skin of a human body since the skin of the human body is weakly acidic, and oxidization of cells inside the body can be efficiently alleviated. In addition, the generated active hydrogen (H⁺) penetrates the skin, reacts with active oxygen (O⁻) inside the skin, and generates moisture to moisten the skin (water retention, moisturization effects are obtained).

The external preparation for skin can be used as cosmetics, pharmaceutical agents, and quasi drugs etc., to be applied to the skin, and its usage mode is not particularly limited. Examples of the usage mode include face lotions, milky lotions, creams, oils, lotions, packs, etc. A specific mode can be, for example, a pack obtained by holding the component on a sheet-like object such as a paper, a nonwoven fabric, or the like. The aforementioned advantageous effects can be easily obtained by hydrating the sheet-like object upon usage and pasting the sheet-like object on the skin or face. In addition, a possible mode is one in which the component and water are separately stored in storage containers that can be kept, and mixed upon usage.

As long as the advantageous effects of the present invention are not compromised, other than the aforementioned components, various components that are generally used in external preparations for skin may be blended in the external preparation for skin according to the present invention as appropriate if necessary. Furthermore, there is no particular limitation in the formulation as long as it can be applied to the skin.

Lastly, although each of the aforementioned embodiments is configured as described above, the present disclosure may comprehensively include the configurations listed below.
1. A bath additive comprising:
   an alkaline agent configured to set a pH of bath water to 7.5 to 9.5; and
   sodium borohydride configured to generate hydrogen in the bath water.
2. An external preparation for skin, the external preparation comprising:
   an alkaline agent configured to set a pH of water to 7.5 to 9.5; and
   sodium borohydride configured to generate hydrogen in the water.
3. A reducing agent for water, the reducing agent comprising:
   an alkaline agent configured to set a pH of water to 7.5 to 9.5; and
   sodium borohydride configured to generate hydrogen in the water.
4. A method for producing a reduced water, the method comprising adding, to water, a reducing agent for water, the reducing agent containing: an alkaline agent configured to set a pH of water to 7.5 to 9.5; and sodium borohydride configured to generate hydrogen in the water.
5. A reduced water comprising:
   an alkaline agent configured to set a pH of water to 7.5 to 9.5; and
   sodium borohydride configured to generate hydrogen in the water.
6. A method for reducing an object, the method comprising
   adding, to water in which a reduction target object is immersed, a reducing agent for water, the agent containing: sodium borohydride configured to generate hydrogen in water; and an alkaline agent configured to set a pH of the water to 7.5 to 9.5, or
   immersing the reduction target object in water having added therein the reducing agent to cause the reduction target object to be in a reduction state.

With any of the aforementioned configurations, reaction of the sodium borohydride is suppressed because of the alkaline agent that sets the pH of the solution (bath water or water) to 7.5 to 9.5, and generation of hydrogen becomes slow. With this, hydrogen, although in a minute amount, is stably generated from the sodium borohydride for a long period of time. In addition, since the generated gaseous hydrogen is produced in a minute amount as fine air bubbles, the gaseous hydrogen is easily dissolved in the water. Thus, generation of hydrogen from the sodium borohydride is sustained for a long period of time, and the oxidation-reduction potential of the water can be maintained in a reduction state for a long period of time because of the dissolved hydrogen. As a result, for example, it becomes possible to alleviate oxidization of cells inside the body of a user.

## Claims

1. A bath additive comprising:
at least one type of a first alkaline agent selected from the group consisting of sodium bicarbonate, ammonium carbonate, and sodium carbonate;
at least one type of a second alkaline agent selected from the group consisting of alkali metal salts or alkaline earth metal salts; and
sodium borohydride as a hydrogen generating agent.

2. The bath additive according to claim 1, wherein the second alkaline agent is at least one type selected from the group consisting of potassium citrate and sodium citrate.

3. The bath additive according to claim 2, further comprising sodium sulfate as an extender.

4. The bath additive according to claim 3, wherein the first alkaline agent is sodium bicarbonate, the second alkaline agent is potassium citrate, the sodium bicarbonate is contained by 35 to 45 wt%, the potassium citrate is contained by 3 to 10 wt%, the sodium sulfate is contained by 50 to 60wt%, and the sodium borohydride is contained by 0.0025 to 10 wt%.

5. An external preparation for skin, the external preparation comprising:
at least one type of a first alkaline agent selected from the group consisting of sodium bicarbonate, ammonium carbonate, and sodium carbonate;
at least one type of a second alkaline agent selected from the group consisting of alkali metal salts or alkaline earth metal salts; and
sodium borohydride as a hydrogen generating agent.

6. The external preparation for skin according to claim 5, wherein the second alkaline agent is at least one type selected from the group consisting of potassium citrate and sodium citrate.

7. The external preparation for skin according to claim 6, further comprising sodium sulfate as an extender.

8. The external preparation for skin according to claim 7, wherein the first alkaline agent is sodium bicarbonate, the second alkaline agent is potassium citrate, the sodium bicarbonate is contained by 35 to 45 wt%, the potassium citrate is contained by 3 to 10 wt%, the sodium sulfate is contained by 50 to 60 wt%, and the sodium borohydride is contained by 0.0025 to 10 wt%.

9. A reducing agent for water, the reducing agent comprising:
at least one type of a first alkaline agent selected from the group consisting of sodium bicarbonate, ammonium carbonate, and sodium carbonate;
at least one type of a second alkaline agent selected from the group consisting of alkali metal salts or alkaline earth metal salts; and
sodium borohydride as a hydrogen generating agent.

10. The reducing agent for water according to claim 9, wherein the second alkaline agent is at least one type selected from the group consisting of potassium citrate and sodium citrate.

11. A method for producing a reduced water, the method comprising adding, to water, a reducing agent containing:
at least one type of a first alkaline agent selected from the group consisting of sodium bicarbonate, ammonium carbonate, and sodium carbonate;
at least one type of a second alkaline agent selected from the group consisting of alkali metal salts or alkaline earth metal salts; and
sodium borohydride as a hydrogen generating agent.

12. The method for producing the reduced water according to claim 11, wherein the second alkaline agent is at least one type selected from the group consisting of potassium citrate and sodium citrate.

13. A reduced water comprising:
at least one type of a first alkaline agent selected from the group consisting of sodium bicarbonate, ammonium carbonate, and sodium carbonate;
at least one type of a second alkaline agent selected from the group consisting of alkali metal salts or alkaline earth metal salts; and
sodium borohydride as a hydrogen generating agent.

14. The reduced water according to claim 13, wherein the second alkaline agent is at least one type selected from the group consisting of potassium citrate and sodium citrate.

15. A method for reducing an object, the method comprising
adding, to water in which a reduction target object is immersed, a reducing agent containing: at least one type of a first alkaline agent selected from the group consisting of sodium bicarbonate, ammonium carbonate, and sodium carbonate; at least one type of a second alkaline agent selected from the group consisting of alkali metal salts or alkaline earth metal salts; and sodium borohydride as a hydrogen generating agent, or
immersing the reduction target object in water having added therein the reducing agent to cause the reduction target object to be in a reduction state.

## Patentansprüche

1. Badezusatz umfassend:
wenigstens eine Art eines ersten alkalischen Mittels ausgewählt aus der Gruppe bestehend aus Natriumhydrogencarbonat, Ammoniumcarbonat und Natriumcarbonat;
wenigstens eine Art eines zweiten alkalischen Mittels ausgewählt aus der Gruppe bestehend aus Alkalimetallsalzen oder Erdalkalimetallsalzen; und
Natriumborhydrid als ein wasserstofferzeugendes Mittel.

2. Badezusatz nach Anspruch 1, bei dem das zweite alkalische Mittel wenigstens eine Art ausgewählt aus der Gruppe bestehend aus Kaliumcitrat und Natriumcitrat ist.

3. Badezusatz nach Anspruch 2 des weiteren umfassend Natriumsulfat als Streckmittel.

4. Badezusatz nach Anspruch 3, bei dem das erste alkalischen Mittel Natriumhydrogencarbonat ist, das zweite alkalischen Mittel Kaliumcitrat ist, das Natriumhydrogencarbonat ist mit 35 bis 45 Gewichts-% enthalten, das Kaliumcitrat ist mit 3 bis 10 Gewichts-% enthalten, das Natriumsulfat ist mit 50 bis 60 Gewichts-% enthalten und das Natriumborhydrid ist mit 0,0025 bis 10 Gewichts-% enthalten.

5. Äußerliches Präparat für die Haut, das äußerlich Präparat umfasst:
wenigstens eine Art eines ersten alkalischen Mittels ausgewählt aus der Gruppe bestehend aus Natriumhydrogencarbonat, Ammoniumcarbonat und Natriumcarbonat;
wenigstens eine Art eines zweiten alkalischen Mittels ausgewählt aus der Gruppe bestehend aus Alkalimetallsalzen oder Erdalkalimetallsalzen; und
Natriumborhydrid als ein wasserstofferzeugendes Mittel.

6. Äußerliches Präparat für die Haut nach Anspruch 5, bei dem das zweite alkalische Mittel wenigstens eine Art ausgewählt aus der Gruppe bestehend aus Kaliumcitrat und Natriumcitrat ist.

7. Äußerliches Präparat für die Haut nach Anspruch 6 des weiteren umfassend Natriumsulfat als Streckmittel.

8. Äußerliches Präparat für die Haut nach Anspruch 7, bei dem das erste alkalischen Mittel Natriumhydrogencarbonat ist, das zweite alkalischen Mittel Kaliumcitrat ist, das Natriumhydrogencarbonat ist mit 35 bis 45 Gewichts-% enthalten, das Kaliumcitrat ist mit 3 bis 10 Gewichts-% enthalten, das Natriumsulfat ist mit 50 bis 60 Gewichts-% enthalten und das Natriumborhydrid ist mit 0,0025 bis 10 Gewichts-% enthalten.

9. Reduktionsmittel für Wasser, das Reduktionsmittel umfasst:
wenigstens eine Art eines ersten alkalischen Mittels ausgewählt aus der Gruppe bestehend aus Natriumhydrogencarbonat, Ammoniumcarbonat und Natriumcarbonat;
wenigstens eine Art eines zweiten alkalischen Mittels ausgewählt aus der Gruppe bestehend aus Alkalimetallsalzen oder Erdalkalimetallsalzen; und
Natriumborhydrid als ein wasserstofferzeugendes Mittel.

10. Reduktionsmittel für Wasser nach Anspruch 9, bei dem das zweite alkalische Mittel wenigstens eine Art ausgewählt aus der Gruppe bestehend aus Kaliumcitrat und Natriumcitrat ist.

11. Verfahren zur Herstellung eines reduzierten Wassers, das Verfahren umfasst die Zugabe eines Reduktionsmittels zu Wasser enthaltend:
wenigstens eine Art eines ersten alkalischen Mittels ausgewählt aus der Gruppe bestehend aus Natriumhydrogencarbonat, Ammoniumcarbonat und Natriumcarbonat;
wenigstens eine Art eines zweiten alkalischen Mittels ausgewählt aus der Gruppe bestehend aus Alkalimetallsalzen oder Erdalkalimetallsalzen; und
Natriumborhydrid als ein wasserstofferzeugendes Mittel.

12. Verfahren zur Herstellung von reduziertem Wasser nach Anspruch 13, bei dem das zweite alkalische Mittel wenigstens eine Art ausgewählt aus der Gruppe bestehend aus Kaliumcitrat und Natriumcitrat ist.

13. Reduziertes Wasser umfassend:
wenigstens eine Art eines ersten alkalischen Mittels ausgewählt aus der Gruppe bestehend aus Natriumhydrogencarbonat, Ammoniumcarbonat und Natriumcarbonat;
wenigstens eine Art eines zweiten alkalischen Mittels ausgewählt aus der Gruppe bestehend aus Alkalimetallsalzen oder Erdalkalimetallsalzen; und
Natriumborhydrid als ein wasserstofferzeugendes Mittel.

14. Reduziertes Wasser nach Anspruch 13, bei dem das zweite alkalische Mittel wenigstens eine Art ausgewählt aus der Gruppe bestehend aus Kaliumcitrat und Natriumcitrat ist.

15. Verfahren zur Reduzierung eines Gegenstandes, das Verfahren umfasst
Zugabe eine Reduktionsmittels zu Wasser, in das ein Trägergegenstand eingetaucht ist, umfassend: wenigstens eine Art eines ersten alkalischen Mittels ausgewählt aus der Gruppe bestehend aus Natriumhydrogencarbonat, Ammoniumcarbonat und Natriumcarbonat; wenigstens eine Art eines zweiten alkalischen Mittels ausgewählt aus der Gruppe bestehend aus Alkalimetallsalzen oder Erdalkalimetallsalzen; und Natriumborhydrid als ein wasserstofferzeugendes Mittel oder
Eintauchen des Reduktionsträgergegenstandes in Wasser, dem das Reduktionsmittel zugegeben wurde, um den Reduktionsträgergegenstand in einem Reduktionszustand zu halten.

## Revendications

1. Additif de bain comprenant :
au moins un type d'un premier agent alcalin choisi dans le groupe composé du bicarbonate de sodium, du carbonate d'ammonium et du carbonate de sodium ;
au moins un type d'un second agent alcalin choisi dans le groupe composé de sels de métaux alcalins ou de sels de métaux alcalino-terreux ; et de borohydrure de sodium comme agent générateur d'hydrogène.

2. Additif de bain selon la revendication 1, dans lequel le second agent alcalin choisi est au moins l'un des agents faisant partie du groupe composé du citrate de potassium et du citrate de sodium.

3. Additif de bain selon la revendication 2, comprenant, en outre, une matière de charge sous la forme de sulfate de sodium.

4. Additif de bain selon la revendication 3, dans lequel le premier agent alcalin est du bicarbonate de sodium, le second agent alcalin est du citrate de potassium, le bicarbonate de sodium étant contenu dans une quantité de 35 à 45 % en poids, le citrate de potassium étant contenu dans une quantité de 3 à 10 % en poids, le sulfate de sodium étant contenu dans une quantité de 50 à 60 % en poids et le borohydrure de sodium étant contenu dans une quantité de 0,0025 à 10 % en poids.

5. Préparation pour la peau à usage externe, la préparation pour usage externe comprenant :
au moins un type d'un premier agent alcalin choisi dans le groupe composé du bicarbonate de sodium, du carbonate d'ammonium et du carbonate de sodium ;
au moins un type d'un second agent alcalin choisi dans le groupe composé de sels de métaux alcalins ou de sels de métaux alcalino-terreux ; et de borohydrure de sodium comme agent générateur d'hydrogène.

6. Préparation pour la peau à usage externe selon la revendication 5, dans laquelle le second agent alcalin choisi est au moins l'un des agents faisant partie du groupe composé du citrate de potassium et du citrate de sodium.

7. Préparation pour la peau à usage externe selon la revendication 6, comprenant en outre une matière de charge sous la forme de sulfate de sodium.

8. Préparation pour la peau à usage externe selon la revendication 7, dans laquelle le premier agent alcalin est du bicarbonate de sodium, le second agent alcalin est du citrate de potassium, le bicarbonate de sodium étant contenu dans une quantité de 35 à 45 % en poids, le citrate de potassium étant contenu dans une quantité de 3 à 10 % en poids, le sulfate de sodium étant contenu dans une quantité de 50 à 60 % en poids et le borohydrure de sodium étant contenu dans une quantité de 0,0025 à 10 % en poids.

9. Agent réducteur pour l'eau, ledit agent réducteur comprenant :
au moins un type d'un premier agent alcalin choisi dans le groupe composé du bicarbonate de sodium, du carbonate d'ammonium et du carbonate de sodium ;
au moins un type d'un second agent alcalin choisi dans le groupe composé de sels de métaux alcalins ou de sels de métaux alcalino-terreux ; et de borohydrure de sodium comme agent générateur d'hydrogène.

10. Agent réducteur pour l'eau selon la revendication 9, dans lequel le second agent alcalin choisi est au moins l'un des agents faisant partie du groupe composé du citrate de potassium et du citrate de sodium.

11. Procédé de production d'eau réduite, ledit procédé comprenant l'adjonction à l'eau d'un agent réducteur contenant :
au moins un type d'un premier agent alcalin choisi dans le groupe composé du bicarbonate de sodium, du carbonate d'ammonium et du carbonate de sodium ;
au moins un type d'un second agent alcalin choisi dans le groupe composé de sels de métaux alcalins ou de sels de métaux alcalino-terreux ; et de borohydrure de sodium comme agent générateur d'hydrogène.

12. Procédé de production d'eau réduite selon la revendication 11, dans lequel le second agent alcalin choisi est au moins l'un des agents faisant partie du groupe composé du citrate de potassium et du citrate de sodium.

13. Eau réduite comprenant :
au moins un type d'un premier agent alcalin choisi dans le groupe composé du bicarbonate de sodium, du carbonate d'ammonium et du carbonate de sodium ;
au moins un type d'un second agent alcalin choisi dans le groupe composé de sels de métaux alcalins ou de sels de métaux alcalino-terreux ; et de borohydrure de sodium comme agent générateur d'hydrogène.

14. Eau réduite selon la revendication 13, dans laquelle le second agent alcalin choisi est au moins l'un des agents faisant partie du groupe composé du citrate de potassium et du citrate de sodium.

15. Procédé de réduction d'un objet, ledit procédé comprenant
l'adjonction à l'eau dans laquelle un objet cible de réduction est immergé, d'un agent réducteur contenant : au moins un type d'un premier agent alcalin choisi dans le groupe composé du bicarbonate de sodium, du carbonate d'ammonium et du carbonate de sodium ; au moins un type d'un second agent alcalin choisi dans le groupe composé de sels de métaux alcalins ou de sels de métaux alcalino-terreux ; et de borohydrure de sodium comme agent générateur d'hydrogène, ou
l'immersion de l'objet cible de réduction dans de l'eau dans laquelle a été ajouté l'agent réducteur afin de provoquer un état de réduction de l'objet cible de la réduction.
